# EUROPÄISCHE PATENTSCHRIFT

(11) **EP 3 452 006 B1**
(45) Veröffentlichungstag und Bekanntmachung des Hinweises auf die Patenterteilung: **20.01.2021**
(21) Anmeldenummer: 16720135.9
(22) Anmeldetag: 03.05.2016
(51) Int. Cl.: A61K 8/34, A61K 8/365, A61K 8/41, A61K 8/73, A61Q 15/00, A61K 8/99

(54) **TRANSPIRATIONSHEMMENDE ZUSAMMENSETZUNG**
ANTIPERSPIRANT COMPOSITION
COMPOSITION ANTITRANSPIRANTE

(43) Veröffentlichungstag der Anmeldung: 13.03.2019
(73) Patentinhaber: Thomas Brunner Hygiene GmbH, 73278 Schlierbach (DE)
(72) Erfinder: BRUNNER, Thomas jr., 73278 Schlierbach (DE)
(74) Vertreter: Patentanwälte Ruff, Wilhelm, Beier, Dauster & Partner mbB
(86) Internationale Anmeldenummer: PCT/EP2016/059934
(87) Internationale Veröffentlichungsnummer: WO 2017/190775

(56) Entgegenhaltungen:
- WO-A1-2016/066801
- DE-A1- 2 703 642
- DE-A1-102014 221 910
- US-A- 3 090 728
- DATABASE GNPD [Online] MINTEL; 1. Mai 2001 (2001-05-01), SSL Healthcare: "Perfumed Deodorant", XP002761301, Database accession no. 98990
- DATABASE GNPD [Online] MINTEL; 1. Juni 2011 (2011-06-01), Natural Alternative: "Natural Deodorant Spray", XP002761302, Database accession no. 1563619
- IP MANUFACTURING: "Antiperspirant Deodorant Roll On", GNPD; MINTEL, 1. August 2014 (2014-08-01), XP002751524, [gefunden am 2014-08-01]

## Beschreibung

### ANWENDUNGSGEBIET UND STAND DER TECHNIK

Die Erfindung betrifft eine transpirationshemmende Zusammensetzung, ein kosmetisches Produkt sowie die Verwendung von Äpfelsäure als transpirationshemmender Wirkstoff bzw. transpirationshemmendes Mittel.

Unter Antitranspirantien werden allgemein Substanzen verstanden, welche die Aktivität von Schweißdrüsen sowie die Menge von abgegebenem Schweiß reduzieren und somit indirekt den Körpergeruch vermindern.

Die Wirkungsweise von handelsüblichen transpirationshemmenden Zusammensetzungen beruht in der Regel auf der Anwesenheit von Aluminiumsalzen, wie beispielsweise Aluminiumchlorhydraten, als transpirationshemmende Wirkstoffe. Die Aluminiumsalze wirken dabei adstringierend und denaturierend. Durch die adstringierende Wirkung kommt es zu einer Verengung der Schweißkanäle. Aufgrund der denaturierenden Wirkung kommt es zu einer Ausfällung von Schweißproteinen unter Ausbildung eines Proteinpfropfs am äußeren Ende der Schweißkanäle der Schweißdrüsen, wodurch insgesamt weniger Schweiß austreten kann.

Transpirationshemmende Zusammensetzungen mit Aluminiumsalzen sind beispielsweise aus den Druckschriften DE 10 2011 086 019 A1, DE 10 2007 063 352 A1, DE 196 42 874 C1, EP 2 481 392 A2, WO 2001/047476 A2, WO 2003/04167 A1, WO 2005/112879 A1 sowie WO 2006/066704 A1 bekannt.

Transpirationshemmende Zusammensetzungen mit einer äpfelsäurehaltigen Titankomplexverbindung sind aus der US 3,090,728 bekannt. Aus der DE 10 2014 221 910 A1 ist ein aluminiumsalzfreies kosmetisches Mittel mit Äpfelsäure als pH-Stellmittel bekannt.

Nachteilig bei den handelsüblichen, aluminiumsalzhaltigen transpirationshemmenden Zusammensetzungen ist, dass die Aluminiumsalze im Verdacht stehen, dauerhafte Schäden im menschlichen Körper zu verursachen. So stehen Aluminiumsalze im Verdacht, Brustkrebs oder Alzheimer auszulösen.

Obwohl wissenschaftliche Belege, welche gesundheitliche Beeinträchtigungen durch aluminiumsalzhaltige Kosmetika zweifelsfrei stützen, bislang nicht zur Verfügung stehen, besteht verbraucherseitig ein Bedarf nach transpirationshemmenden Zusammensetzungen, welche keinen medizinischen Bedenken begegnen.

### AUFGABE UND LÖSUNG

Der vorliegenden Erfindung liegt daher die Aufgabe zugrunde, eine transpirationshemmende Zusammensetzung sowie ein kosmetisches Produkt bereitzustellen, welche jeweils den oben genannten Bedarf adressieren und aus dem Stand der Technik bekannte Nachteile vermeiden. Diese Aufgabe wird gelöst durch eine transpirationshemmende Zusammensetzung mit den Merkmalen des Anspruchs 1 sowie durch ein kosmetisches Produkt gemäß Anspruch 11. Bevorzugte Ausführungsformen sind in den abhängigen Ansprüchen 2 bis 10 definiert. Der Wortlaut sämtlicher Ansprüche wird hiermit durch ausdrückliche Bezugnahme zum Inhalt der vorliegenden Beschreibung gemacht.

Gemäß einem ersten Aspekt betrifft die Erfindung eine transpirations- bzw. schweißhemmende Zusamensetzung, gemäss Anspruch 1.

Die Zusammensetzung zeichnet sich besonders dadurch aus, dass es sich bei dem transpirationshemmenden Wirkstoff um Äpfelsäure (2-Hydroxybernsteinsäure bzw. 2-Hydroxybutan-1,4-disäure, auch als Apfelsäure bezeichnet) handelt.

Unter dem Ausdruck "transpirationshemmende Zusammensetzung" soll im Sinne der vorliegenden Erfindung eine vorzugsweise kosmetische Zusammensetzung verstanden werden, welche eine reduzierte Aktivität von Schweißdrüsen, insbesondere von apokrinen Schweißdrüsen, sowie eine reduzierte Schweißabsonderung bewirkt. Entsprechend soll unter dem Ausdruck "transpirationshemmender Wirkstoff, im Sinne der vorliegenden Erfindung ein Wirkstoff verstanden werden, welcher die Aktivität von Schweißdrüsen, insbesondere apokrinen Schweißdrüsen, sowie die Schweißabsonderung reduziert. Der transpirationshemmende Wirkstoff kann im Sinne der vorliegenden Erfindung daher auch als Schweißhemmer bzw. Antitranspirant bezeichnet werden. Bei den apokrinen Schweißdrüsen handelt es sich vorzugsweise um solche, welche im Bereich der Axilla (pyramidenförmiger anatomischer Raum zwischen der lateralen Brustwand und dem medialen Oberarm), der Brustwarze, der Leistengegend, der perianalen Region (Glandulae circumanales) und der Schamregion, insbesondere im Bereich der weiblichen großen Schamlippen (Mons pubis) sowie des männlichen Hodensacks, lokalisiert sind.

Der Ausdruck "Äpfelsäure" kann im Sinne der vorliegenden Erfindung die Säure als solche, d.h. 2-Hydroxybernsteinsäure bzw. 2-Hydroxybutan-1,4-disäure, und/oder ein Salz davon, d.h. ein Malat, bedeuten. Bei dem Salz kann es sich beispielsweise um Mono- oder Dinatriummalat handeln. Bevorzugt bedeutet der Ausdruck "Äpfelsäure" im Sinne der vorliegenden Erfindung jedoch die Säure als solche.

Der Erfindung liegt die überraschende Erkenntnis zugrunde, dass Äpfelsäure eine transpirationshemmende Wirkung besitzt und insbesondere zum Ersatz von aluminiumhaltigen Antitranspirantien (d.h. von aluminiumhaltigen, transpirationshemmenden Wirkstoffen), vorzugsweise von Aluminiumsalzen, in kosmetischen Zusammensetzungen verwendet werden kann. Die transpirationshemmende Wirkung von Äpfelsäure beruht - wie bei herkömmlichen Aluminiumsalzen - darauf, dass Äpfelsäure, insbesondere in den nachfolgend beschriebenen Anteilen, eine denaturierende Wirkung in Bezug auf Proteinbestandteile des Schweißes besitzt. Die denaturierende Wirkung der Äpfelsäure bewirkt die Ausfällung der Proteine. Dadurch kommt es zur Ausbildung eines Proteinpfropfs, welcher die äußeren Enden der Schweißkanäle blockiert, wodurch insgesamt weniger Schweiß austreten kann.

In einer bevorzugten Ausführungsform ist die transpirationshemmende Zusammensetzung frei von Salzen, welche ausgewählt sind aus der Gruppe bestehend aus Zirkoniumsalze, Zinksalze und Mischsalze davon. Mit anderen Worten ist es erfindungsgemäß bevorzugt, wenn die Zusammensetzung zirkonium- und/oder zinksalzfrei ist.

Der Ausdruck "Aluminiumsalz" definiert im Sinne der vorliegenden Erfindung allgemein aluminiumhaltige Salze und umfasst mithin sowohl anorganische als auch organische Salze des Aluminiums, wie beispielsweise Aluminiumchlorhydrate und Aluminiumhydroxylaktate, sowie auch Mischsalze des Aluminiums mit anderen Metallelementen, wie beispielsweise Zirkonium.

Der Ausdruck "Zirkoniumsalz" definiert im Sinne der vorliegenden Erfindung allgemein zirkoniumhaltige Salze und umfasst mithin sowohl anorganische als auch organische Salze des Zirkoniums sowie auch Mischsalze des Zirkoniums mit anderen Metallelementen, wie beispielsweise Aluminium.

Der Ausdruck "Zinksalz" definiert im Sinne der vorliegenden Erfindung allgemein zinkhaltige Salze und umfasst mithin sowohl anorganische als auch organische Salze des Zinks sowie auch Mischsalze des Zinks mit anderen Metallelementen.

Bei der transpirationshemmenden Zusammensetzung handelt es sich um eine aluminiumsalzfreie, transpirationshemmende kosmetische Zusammensetzung, das heißt um eine transpirationshemmende Zusammensetzung, welche frei von Aluminiumsalzen ist.

In einer weiteren Ausführungsform ist die transpirationshemmende Zusammensetzung frei von einem aluminiumhaltigen, transpirationshemmenden Wirkstoff. Der Ausdruck "aluminiumhaltiger, transpirationshemmender Wirkstoff" bedeutet im Sinne der vorliegenden Erfindung allgemein eine aluminiumhaltige Substanz bzw. Verbindung, welche transpirationshemmende Eigenschaften besitzt. Bei einer solchen Substanz bzw. Verbindung kann es sich somit nicht nur um ein Aluminiumsalz, sondern beispielsweise auch um eine kovalente Aluminiumverbindung oder aluminiumhaltige Komplexverbindung, wie beispielsweise Aluminium-Zirkonium-Tetrachlorohydrex-Glycin-Komplex, handeln.

In einer weiteren Ausführungsform ist die transpirationshemmende Zusammensetzung frei von einem zirkoniumhaltigen, transpirationshemmenden Wirkstoff. Der Ausdruck "zirkoniumhaltiger, transpirationshemmender Wirkstoff" bedeutet im Sinne der vorliegenden Erfindung allgemein eine zirkoniumhaltige Substanz bzw. Verbindung, welche transpirationshemmende Eigenschaften besitzt. Bei einer solchen Substanz bzw. Verbindung kann es sich somit nicht nur um ein Zirkoniumsalz, sondern beispielsweise auch um eine kovalente Zirkoniumverbindung oder eine zirkoniumhaltige Komplexverbindung handeln.

In einer weiteren Ausführungsform ist die transpirationshemmende Zusammensetzung frei von einem zinkhaltigen, transpirationshemmenden Wirkstoff. Der Ausdruck "zinkhaltiger, transpirationshemmender Wirkstoff" bedeutet im Sinne der vorliegenden Erfindung allgemein eine zinkhaltige Substanz bzw. Verbindung, welche transpirationshemmende Eigenschaften besitzt. Eine derartige Substanz bzw. Verbindung kann nicht nur ein Zinksalz, sondern beispielsweise auch eine kovalente Zinkverbindung oder eine zinkhaltige Komplexverbindung bedeuten.

In einer weiteren Ausführungsform weist die Äpfelsäure einen Anteil von 1 Gew.-% bis 10 Gew.-%, insbesondere 2 Gew.-% bis 8 Gew.-%, vorzugsweise 3 Gew.-% bis 5 Gew.-%, auf, bezogen auf das Gesamtgewicht der Zusammensetzung. Der Erfindung liegt weiterhin die überraschende Erkenntnis zugrunde, dass die denaturierenden Eigenschaften der Äpfelsäure bei den in diesem Absatz genannten Anteilen besonders stark zur Geltung kommen. Ein weiterer Vorteil der in diesem Absatz beschriebenen Äpfelsäureanteile besteht darin, dass sie den Zusatz von Konservierungsstoffen entbehrlich machen können.

In einer weiteren Ausführungsform handelt es sich bei der Äpfelsäure um D-(+)-Äpfelsäure.

In einer weiteren Ausführungsform handelt es sich bei der Äpfelsäure um L-(-)-Äpfelsäure.

In einer weiteren Ausführungsform liegt die Äpfelsäure in Form einer Mischung, insbesondere in Form einer racemischen Mischung, aus D-(+)-Äpfelsäure und L-(-)-Äpfelsäure vor.

In einer weiteren Ausführungsform weist die Zusammensetzung ferner eine weitere Hydroxycarbonsäure, insbesondere ausgewählt aus der Gruppe bestehend aus Weinsäure, Zitronensäure, Gallussäure und Mischungen aus zwei oder mehreren der genannten Hydroxycarbonsäuren, auf.

Die weitere Hydroxycarbonsäure kann insbesondere einen Anteil von 1 Gew.-% bis 11 Gew.-%, insbesondere 2 Gew.-% bis 8 Gew.-%, bevorzugt 3 Gew.-% bis 6.5 Gew.-%, aufweisen, bezogen auf das Gesamtgewicht der Zusammensetzung

In einer weiteren Ausführungsform ist Triethanolamin als pH-Regulator ausgenommen. Mit anderen Worten ist die transpirationshemmende Zusammensetzung gemäß einer weiteren Ausführungsform frei von Triethanolamin, d.h. triethanolaminfrei.

Bei dem pH-Regulator handelt es sich in einer weiteren Ausführungsform um eine gegenüber der Äpfelsäure inerte Verbindung.

Durch die Verwendung eines pH-Regulators, insbesondere in den nachfolgend angegebenen Anteilen, können mit besonderem Vorteil durch die Äpfelsäure verursachte Hautirritationen und andere Hautschädigungen vermieden werden.

Der pH-Regulator weist vorzugsweise einen Anteil von 0.25 Gew.-% bis 5 Gew.-%, bevorzugt 0.5 Gew.-% bis 4 Gew.-%, besonders bevorzugt 0.75 Gew.-% bis 1.75 Gew.-%, auf, bezogen auf das Gesamtgewicht der Zusammensetzung.

In einer weiteren Ausführungsform ist der pH-Regulator ausgewählt aus der Gruppe bestehend aus Monoethanolamin, Diethanolamin und ein Gemischaus Monoethanolamin und Diethanolamin. Monoethanolamin ist erfindungsgemäß besonders bevorzugt.

Die transpirationshemmende Zusammensetzung weist in einer weiteren Ausführungsform einen pH-Wert von 3 bis 6.5, insbesondere 3.5 bis 6, vorzugsweise 4 bis 5, auf.

Bei dem flüssigen Träger handelt es sich um einen Alkohol oder ein Alkoholgemisch. Der Alkohol ist ausgewählt aus der Gruppe bestehend aus Ethanol, Isopropanol, 2-Methyl-propanol-2 und Gemische aus zwei oder mehreren der genannten Alkohole. Besonders bevorzugt handelt es sich bei dem flüssigen Träger um Ethanol.

Insbesondere kann es sich bei dem flüssigen Träger um ein Gemisch aus Ethanol, Isopropanol und 2-Methyl-propanol-2 handeln. Beispielsweise kann es sich bei dem flüssigen Träger um ein Gemisch aus Ethanol, Isopropanol und 2-Methyl-propanol-2 handeln, wobei das Gemisch einen Ethanolanteil von 96 Gew.-%, einen Isopropanolanteil von 2 Gew.-% sowie einen Anteil an 2-Methyl-propanol-2 von 2 Gew.-% aufweist, jeweils bezogen auf das Gesamtgewicht des Gemisches. Ein derartiges Gemisch ist unter dem Handelsnamen Cosmetol (96%ig) kommerziell erhältlich.

Die Verwendung von Ethanol als flüssiger Träger ist besonders vorteilhaft, da Ethanol, insbesondere in den im Folgenden offenbarten Anteilen, stark denaturierend auf Proteinbausteine wirkt, ohne Hautirritationen oder andere unerwünschte Nebenwirkungen zu verursachen. Hierdurch lassen sich die transpirationshemmenden Eigenschaften der erfindungsgemäßen Zusammensetzung zusätzlich verstärken. Außerdem wirkt Ethanol, insbesondere in den im Folgenden offenbarten Anteilen, antibakteriell, wodurch auf der Haut befindliche Bakterien, welche den Schweiß zersetzen und deren Ausscheidungen ursächlich für Körper- bzw. Schweißgeruch sind, abgetötet werden können. Auf diese Weise können die körper- bzw. schweißgeruchsbekämpfenden Eigenschaften der Zusammensetzung zusätzlich verstärkt werden. Ein weiterer Vorteil besteht darin, dass auch bei der Verwendung von Ethanol der Zusatz von Konservierungsstoffen entbehrlich sein kann.

In einer bevorzugten Ausführungsform weist der flüssige Träger einen Anteil von höchstens 50 Gew.-% auf, bezogen auf das Gesamtgewicht der Zusammensetzung. Der flüssige Träger kann insbesondere einen Anteil von 20 Gew.-% bis 50 Gew.-%, bevorzugt 25 Gew.-% bis 45 Gew.-%, besonders bevorzugt 30 Gew.-% bis 40 Gew.-%, aufweisen, bezogen auf das Gesamtgewicht der Zusammensetzung.

Besonders überraschend war die Erkenntnis, dass Ethanol als flüssiger Träger, insbesondere in den oben angegebenen Anteilen, zusammen mit der Äpfelsäure eine Art synergistischen Antitranspirant-Wirkkomplex bildet, dessen transpirationshemmende Wirkung mit derjenigen einer wasserbasierten, aluminiumsalzhaltigen Kosmetik-Formulierung vergleichbar ist.

In einer weiteren Ausführungsform weist das Wasser einen Anteil von 10 Gew.-% bis 80 Gew.-%, insbesondere 20 Gew.-% bis 75 Gew.-%, vorzugsweise 30 Gew.-% bis 65 Gew.-%, auf, bezogen auf das Gesamtgewicht der Zusammensetzung.

In einer weiteren Ausführungsform enthält die transpirationshemmende Zusammensetzung ferner Saccharomyces-Ferment. Hierbei handelt es sich um ein Endprodukt der Fermentation durch Saccharomyces, das heißt um ein Zuckerhefen-Ferment. Ein derartiges Ferment ist beispielsweise unter dem Handelsnamen Deosent, DeoPlex Clear H5630 kommerziell erhältlich. Saccharomyces-Ferment besitzt mit besonderem Vorteil eine geruchsüberdeckende bzw. geruchsmindernde Wirkung. Diese Wirkung beruht auf einer enzymatischen Umsetzung bestimmter Riechstoffgruppen, wodurch entsprechende Geruchs- bzw. Duftstoffe zu größeren, schweren Verbindungen umgesetzt werden, welche aufgrund ihres höheren Gewichts nicht mehr so leicht verdampfen können und daher nicht mehr geruchlich wahrgenommen werden.

Vorzugsweise weist das Saccharomyces-Ferment einen Anteil von 0.5 Gew.-% bis 5 Gew.-%, insbesondere 1 Gew.-% bis 3.5 Gew.-%, vorzugsweise 1.25 Gew.-% bis 2.5 Gew.-%, auf, bezogen auf das Gesamtgewicht der Zusammensetzung.

In einer weiteren Ausführungsform enthält die transpirationshemmende Zusammensetzung ferner einen Komplexbildner. Bevorzugt handelt es sich bei dem Komplexbildner um ein Polyphosphat. Beispielsweise kann der Komplexbildner ausgewählt sein aus der Gruppe bestehend aus Natriumpolyphosphat, Natrium-Hexametaphosphat, Kaliumpolyphosphat, Calciumpolyphosphat, Natriumcalciumpolyphosphat und Mischungen aus zwei der mehreren der genannten Polyphosphate.

Bevorzugt weist der Komplexbildner einen Anteil von 0.25 Gew.-% bis 5 Gew.-%, insbesondere 0.5 Gew.-% bis 4 Gew.-%, vorzugsweise 0.75 Gew.-% bis 3 Gew.-%, auf, bezogen auf das Gesamtgewicht der Zusammensetzung.

In einer weiteren Ausführungsform enthält die transpirationshemmende Zusammensetzung ferner einen Viskositätsregulator bzw. Filmbildner. Der Viskositätsregulator bzw. Filmbildner kann beispielsweise ausgewählt sein aus der Gruppe bestehend aus Methylhydroxyethylcellulose, Carboxymethylcellulose, Methylcellulose, Ethylcellulose, Hydroxyethylcellulose, Hydroxyethylmethylcellulose, Hydroxypropylmethylcellulose, homopolymere Polyacrylsäure (sogenanntes Carbomer), heteropolymere Polyacrylsäure bzw. Acrylat-Einheiten enthaltende Copolymere, Guaran, Guar Gummi, Hydroxypropyl Guar (Guar Gummi, 2-Hydroxypropylether, depolymerisiert) wie Esaflor® HDR, Carboxymethyl Guar und Mischungen aus zwei oder mehreren der genannten Viskositätsregulatoren bzw. Filmbildner.

Bevorzugt weist der Viskositätsregulator bzw. Filmbildner einen Anteil von 0.01 Gew.-% bis 1 Gew.-%, insbesondere 0.05 Gew.-% bis 0.75 Gew.-%, bevorzugt 0.1 Gew.-% bis 0.5 Gew.-%, auf, bezogen auf das Gesamtgewicht der Zusammensetzung.

Um eine verbesserte Durchdringung der Hautbarriere, das heißt eine verbesserte dermale Aufnahme, zu erzielen, enthält die transpirationshemmende Zusammensetzung in einer weiteren Ausführungsform ferner ein Tensid, insbesondere ein emulgierendes Tensid.

Das Tensid kann beispielsweise ausgewählt sein aus der Gruppe bestehend aus Polyoxyethylen(20)-sorbitan-monolaurat, Kokosglucoside wie Plantacare 818, Cocamidopropylbetain, Coco-Betaine wie Dehyton PK 45 und/oder Tego Betain F50 und Mischungen aus zwei oder mehreren der genannten Tenside. Polyoxyethylen(20)-sorbitan-monolaurat ist beispielsweise unter dem Handelsnamen Polysorbat 20 kommerziell erhältlich.

Bevorzugt weist das Tensid einen Anteil von 0.1 Gew.-% bis 2.5 Gew.-%, insbesondere 0.25 Gew.-% bis 2 Gew.-%, bevorzugt 0.5 Gew.-% bis 1.5 Gew.-%, auf, bezogen auf das Gesamtgewicht der Zusammensetzung.

In einer unter Hautpflegegesichtspunkten vorteilhaften Ausführungsform enthält die transpirationshemmende Zusammensetzung ferner wenigstens einen Hautpflegestoff, insbesondere ein Feuchthalte- und/oder Hautbefeuchtungsmittel.

Der wenigstens eine Hautpflegestoff kann beispielsweise ausgewählt sein aus der Gruppe bestehend aus Aminosäuren, Milchsäure und deren Salze, Lactitol, Harnstoff, Harnstoffderivate, Harnsäure, Glucosamin, Kreatinin, Spaltprodukte des Collagens, Chitosan, Chitosansalze, Chitosanderivate, Polyole wie Glycerin und/oder Diglycerin, Polyolderivate, Allantoin, Dexpanthenol, Triglycerin, Glycerinester wie Glycerincaprylat, Ethylenglykol, Propylenglykol, Butylenglykol, Erythrit, 1,2,6-Hexantriol, Polyethylenglykole wie PEG-4, PEG-6, PEG-7, PEG-8, PEG-9, PEG-10, PEG-12, PEG-14, PEG-16, PEG-18, PEG-20, Zucker und Zuckerderivate wie Fructose, Glucose, Maltose, Maltit, Mannit, Inosit, Sorbit, Sorbitylsilandiol, Sucrose, Trehalose, Xylose, Xylit, Glucoronsäure und/oder Salze davon und Mischungen, insbesondere Hautpflegestoffkomplexe, aus wenigstens zwei der genannten Hautpflegestoffe.

Bevorzugt ist der wenigstens eine Hautpflegestoff ausgewählt aus der Gruppe bestehend aus Harnstoff, Propan-1,2-diol, Glycerin, Diglycerin, Allantoin, Dexpanthenol und Mischungen, insbesondere Hautpflegestoffkomplexe, aus wenigstens zwei der genannten Hautpflegestoffe.

Der wenigstens eine Hautpflegestoff, insbesondere ein Hautpflegestoffkomplex aus wenigstens zwei der oben genannten Hautpflegestoffe, weist vorzugsweise einen Anteil von 2 Gew.-% bis 25 Gew.-%, insbesondere 3.5 Gew.-% bis 20.5 Gew.-%, bevorzugt 4.5 Gew.-% bis 18.5 Gew.-%, auf, bezogen auf das Gesamtgewicht der Zusammensetzung.

Die transpirationshemmende Zusammensetzung kann insbesondere einen Harnstoffanteil von 0.1 Gew.-% bis 3 Gew.-%, insbesondere 0.2 Gew.-% bis 2 Gew.-%, vorzugsweise 0.4 Gew.-% bis 1 Gew.-%, aufweisen, bezogen auf das Gesamtgewicht der Zusammensetzung.

In einer weiteren Ausführungsform weist die transpirationshemmende Zusammensetzung einen Anteil an Propan-1,2-diol von 0.5 Gew.-% bis 5 Gew.-%, insbesondere 0.75 Gew.-% bis 4 Gew.-%, bevorzugt 1 Gew.-% bis 3 Gew.-%, auf, bezogen auf das Gesamtgewicht der Zusammensetzung.

In einer weiteren Ausführungsform weist die transpirationshemmende Zusammensetzung einen Anteil an Glycerin von 1 Gew.-% bis 12 Gew.-%, insbesondere 2 Gew.-% bis 10 Gew.-%, bevorzugt 2,5 Gew.-% bis 8 Gew.-%, auf, bezogen auf das Gesamtgewicht der Zusammensetzung.

In einer weiteren Ausführungsform weist die transpirationshemmende Zusammensetzung einen Anteil an Allantoin von 0.05 Gew.-% bis 0.5 Gew.-%, insbesondere 0.1 Gew.-% bis 0.4 Gew.-%, bevorzugt 0.15 Gew.-% bis 0.3 Gew.-%, auf, bezogen auf das Gesamtgewicht der Zusammensetzung.

In einer weiteren Ausführungsform weist die transpirationshemmende Zusammensetzung einen Anteil an Dexpanthenol von 0.25 Gew.-% bis 5 Gew.-%, insbesondere 0.5Gew.-% bis 4 Gew.-%, bevorzugt 0.75 Gew.-% bis 3 Gew.-%, auf, bezogen auf das Gesamtgewicht der Zusammensetzung.

In einer unter duftneutralisierenden Gesichtspunkten vorteilhaften Ausführungsform enthält die transpirationshemmende Zusammensetzung ferner einen desodierenden Wirkstoff. Der desodierende Wirkstoff kann beispielsweise ausgewählt sein aus der Gruppe bestehend aus Duft- bzw. Geruchsstoffe, Parfüm (flüssiges Gemisch aus Alkohol, insbesondere Ethanol, und Geruchs- bzw. Duftstoffen), Geruchsabsorber oder -löscher, antimikrobielle Wirkstoffe, Enzyminhibitoren und Mischungen aus zwei oder mehreren der genannten desodierenden Wirkstoffen.

Beispielsweise kann die transpirationshemmende Zusammensetzung ferner einen Duft- bzw. Geruchsstoff enthalten. Hinsichtlich des Duft- bzw. Geruchsstoffes bestehen grundsätzlich keinerlei Bedenken, solange es sich hierbei vorzugsweise um einen Duft- bzw. Geruchsstoff handelt, welcher eine leicht saure Umgebung toleriert. Beispielsweise kann es sich bei dem Duft- bzw. Geruchsstoff um Menthol handeln.

Bevorzugt weist der Duft- bzw. Geruchsstoff einen Anteil von 0.05 Gew.-% bis 2.5 Gew.-%, insbesondere 0.1 Gew.-% bis 1.5 Gew.-%, bevorzugt 0.2 Gew.-% bis 1 Gew.-%, auf, bezogen auf das Gesamtgewicht der Zusammensetzung.

Bei den oben erwähnten Geruchsabsorber bzw. -löscher kann es sich beispielsweise um Silikate, insbesondere Schichtsilikate, wie beispielsweise Montmorillonit, Kaolinit, Beidellit, Saponit, Bentonit, Zeolithe, Cyclodextrine oder Mischungen aus zwei oder mehreren der genannten Geruchsabsorber bzw. -löscher, handeln.

Die oben erwähnten antimikrobiellen Wirkstoffe können beispielsweise ausgewählt sein aus der Gruppe bestehend aus Triclosan, Chlorhexidin, Chlorhexidingluconat, Bromchlorophen, Dichlorophen, Chlorothymol, Chloroxylenol, Hexachlorophen, Ammoniumphenolsulfonat, Benzalkoniumhalogenide, Benzalkoniumcetylphosphat, Benzalkoniumsaccharinat, Benzethoniumchlorid, Cetylpyridiniumchlorid, Laurylpyridiniumchlorid, Laurylisoquinoliniumbromid, Methylbenzethoniumchlorid, Terpenalkohole wie beispielsweise Farnesol, Chlorophyllin-Kupfer-Komplexe, Carbonsäureester von Mono-, Di- und/oder Triglycerin wie Glycerinmonolaurat und/oder Diglycerinmonocaprinat, Fettsäuren, Pflanzenextrakte wie Grüner Tee und/oder Bestandteil des Lindenblütenöls, und Mischungen aus zwei oder mehreren der genannten antimikrobiellen Wirkstoffe.

In einer weiteren Ausführungsform enthält die transpirationshemmende Zusammensetzung ferner einen Konservierungsstoff. Der Konservierungsstoff kann beispielsweise ausgewählt sein aus der Gruppe bestehend aus Benzoesäure und deren Derivate, Propionsäure und deren Derivate, Salicylsäure und deren Derivate, Alkohole und deren Derivate, Guajacol und dessen Derivate, Hydantoin und dessen Derivate, Ferulasäure und deren Derivate, Sorbinsäure und deren Derivate und Mischungen aus zwei oder mehreren der genannten Konservierungsstoffe.

Der Konservierungsstoff kann insbesondere einen Anteil von 0.05 Gew.-% bis 1 Gew.-%, insbesondere 0.1 Gew.-% bis 0.75 Gew.-%, bevorzugt 0.2 Gew.-% bis 0.5 Gew.-%, aufweisen, bezogen auf das Gesamtgewicht der Zusammensetzung.

In einer weiteren Ausführungsform ist die transpirationshemmende Zusammensetzung frei von einem Konservierungsstoff. Wie bereits erwähnt, kann aufgrund der Verwendung von insbesondere Ethanol als flüssigem Träger und/oder durch die Verwendung von Äpfelsäure der Zusatz von Konservierungsstoffen entbehrlich sein.

In einer weiteren Ausführungsform enthält die transpirationshemmende Zusammensetzung ferner ein Antioxidans.

Das Antioxidans kann beispielsweise ausgewählt sein aus der Gruppe bestehend aus Aminosäuren wie Glycin, Histidin, Tyrosin und/oder Tryptophan, Aminosäurederivate, Imidazol, Imidazolderivate wie Urocaninsäure, Carotinoide, Carotine wie α-Carotin, β-Carotin und/oder Lycopin, Carotinoid-Derivate, Carotin-Derivate, Liponsäure und deren Derivate wie Dihydroliponsäure, Thioverbindungen, Huminsäuren, Gallensäure, Gallenextrakte, Flavonoide, Katechine, Bilirubin, Biliverdin, ungesättigte Fettsäuren und deren Derivate wie γ-Linolensäure, Linolsäure, Arachidonsäure, Ölsäure, Folsäure und deren Derivate, Hydrochinon und dessen Derivate, Ubichinon und Ubichinol sowie deren Derivate, Vitamin C und dessen Derivate, Tocopherole und deren Derivate und Mischungen aus zwei oder mehreren der genannten Antioxidantien.

Das Antioxidans kann insbesondere einen Anteil von 0.05 Gew.-% bis 1 Gew.-%, insbesondere 0.1 Gew.-% bis 0.75 Gew.-%, bevorzugt 0.2 Gew.-% bis 0.5 Gew.-%, aufweisen, bezogen auf das Gesamtgewicht der Zusammensetzung.

In einer weiteren Ausführungsform enthält die transpirationshemmende Zusammensetzung ferner eine Zinkverbindung, insbesondere ein Zinksalz, wie beispielsweise Zinkoxid.

In einer weiteren Ausführungsform enthält die transpirationshemmende Zusammensetzung ferner eine antiadhäsive Verbindung. Bei der antiadhäsiven Verbindung kann es sich beispielsweise um eine silikonbasierte Verbindung, insbesondere um ein Cyclopentasiloxan, handeln.

In einer weiteren Ausführungsform enthält die transpirationshemmende Zusammensetzung auf 100 Gew.-% folgende Inhaltsstoffe:
- 1 Gew.-% bis 11 Gew.-%, insbesondere 2 Gew.-% bis 8 Gew.-%, vorzugsweise 3 Gew.-% bis 6.5 Gew.-%, Apfelsäure,
- 0.25 Gew.-% bis 5 Gew.-%, insbesondere 0.5 Gew.-% bis 4 Gew.-%, vorzugsweise 0.75 Gew.-% bis 1.75 Gew.-%, eines pH-Regulators,
- 20 Gew.-% bis 50 Gew.-%, insbesondere 25 Gew.-% bis 45 Gew.-%, vorzugsweise 30 Gew.-% bis 40 Gew.-%, des flüssigen Trägers,
- 10 Gew.-% bis 80 Gew.-%, insbesondere 20 Gew.-% bis 75 Gew.-%, vorzugsweise 30 Gew.-% bis 65 Gew.-%, Wasser.
- 0.5 Gew.-% bis 5 Gew.-%, insbesondere 1 Gew.-% bis 3.5 Gew.-%, vorzugsweise 1.25 Gew.-% bis 2.5 Gew.-%, Saccharomyces Ferment
- 0.25 Gew.-% bis 5 Gew.-%, insbesondere 0.5 Gew.-% bis 4 Gew.-%, vorzugsweise 0.75 Gew.-% bis 3 Gew.-%, eines Komplexbildners,
- 0.01 Gew.-% bis 1 Gew.-%, insbesondere 0.05 Gew.-% bis 0.75 Gew.-%, vorzugsweise 0.1 Gew.-% bis 0.5 Gew.-%, eines Viskositätsregulators bzw. Filmbildners,
- 0.1 Gew.-% bis 2.5 Gew.-%, insbesondere 0.25 Gew.-% bis 2 Gew.-%, vorzugsweise 0.5 Gew.-% bis 1.5 Gew.-%, eines Tensids
- 2 Gew.-% bis 25 Gew.-%, insbesondere 3.5 Gew.-% bis 20.5 Gew.-%, vorzugsweise 4.5 Gew.-% bis 18.5 Gew.-%, eines Hautpflegestoff-Komplexes,
- 0.05 Gew.-% bis 2.5 Gew.-%, insbesondere 0.1 Gew.-% bis 1.5 Gew.-%, vorzugsweise 0.2 Gew.-% bis 1 Gew.-%, eines Duftstoffes.

In einer weiteren Ausführungsform besteht die transpirationshemmende Zusammensetzung aus Äpfelsäure, einem pH-Regulator, einem flüssigen Träger, Wasser und gegebenenfalls wenigstens einem weiteren Inhaltsstoff, welcher vorzugsweise ausgewählt ist aus der Gruppe bestehend aus Saccharomyces-Ferment, Komplexbildner, Viskositätsregulator bzw. Filmbildner, Tensid, Hautpflegestoff, Konservierungsstoff, Antioxidans, Zinkverbindung, antiadhäsive Verbindung und Mischungen aus zwei oder mehreren der genannten weiteren Inhaltsstoffe. Bezüglich weiterer Merkmale und Vorteile der Äpfelsäure, des pH-Regulators, des flüssigen Trägers, des Wassers sowie des gegebenenfalls wenigstens einen weiteren Inhaltsstoffes wird vollständig auf die bisherige Beschreibung Bezug genommen.

Die erfindungsgemäße Zusammensetzung kann insbesondere aus Äpfelsäure, einem pH-Regulator, einem flüssigen Träger, Wasser, einem Komplexbildner, einem Viskositätsregulator bzw. Filmbildner, einem Tensid, einem Hautpflegestoff, einem Duft- bzw. Geruchsstoff und gegebenenfalls wenigstens einem weiteren Inhaltsstoff bestehen, welcher vorzugsweise ausgewählt ist aus der Gruppe bestehend aus Saccharomyces-Ferment, einer antiadhäsiven Verbindung, einer Zinkverbindung und Mischungen aus zwei oder mehreren der genannten weiteren Inhaltsstoffe. Bezüglich weiterer Merkmale und Vorteile der Äpfelsäure, des pH-Regulators, des flüssigen Trägers, des Wassers, des Komplexbildners, des Viskositätsregulators bzw. Filmbildners, des Tensids, des Hautpflegestoffes, des Duft- bzw. Geruchsstoffes sowie des gegebenenfalls wenigstens einen weiteren Inhaltsstoffes wird zur Vermeidung von Wiederholungen ebenfalls vollständig auf die bisherige Beschreibung Bezug genommen.

In einer weiteren Ausführungsform ist die transpirationshemmende Zusammensetzung als Lösung, Suspension, Gel wie Hydrogel, Creme, Paste, Salbe, Roll-on-Zusammensetzung, Stift, Spray-Sud, Pumpspray oder zusammen mit einem Treibmittel als Aerosolspray konfektioniert. Mit anderen Worten ist es erfindungsgemäß bevorzugt, wenn die transpirationshemmende Zusammensetzung in Form einer Lösung, einer Suspension, eines Gels wie Hydrogels, einer Creme, einer Paste, einer Salbe, einer Roll-on-Zusammensetzung, eines Stifts, eines Spray-Suds, eines Pumpsprays oder zusammen mit einem Treibmittel in Form eines Aerosolsprays vorliegt.

Bei der erfindungsgemäßen Zusammensetzung handelt es sich um eine transpirationshemmende kosmetische Zusammensetzung.

Gemäß einem zweiten Aspekt betrifft die Erfindung ein kosmetisches Produkt, welches eine transpirationshemmende Zusammensetzung gemäß erstem Erfindungsaspekt enthält oder aus einer solchen Zusammensetzung besteht oder hergestellt ist.

Bei dem kosmetischen Produkt handelt es sich vorzugsweise um ein kosmetisches Gel wie kosmetisches Hydrogel, eine kosmetische Creme, eine kosmetische Paste, eine kosmetische Salbe, ein kosmetisches Roll-on-Produkt, einen kosmetischen Stift, ein kosmetisches Spray-Sud, ein kosmetisches Pumpspray oder ein kosmetisches Aerosolspray. Im Falle einer Ausgestaltung als kosmetisches Aerosolspray enthält das Produkt neben einer transpirationshemmenden Zusammensetzung gemäß erstem Erfindungsaspekt vorzugsweise ein geeignetes Treibmittel, wie beispielsweiseButangas, Isobutangas oder Popangas.

Bezüglich weiterer Merkmale und Vorteile des kosmetischen Produkts, insbesondere der transpirationshemmenden Zusammensetzung, wird vollständig auf die im Rahmen des ersten Erfindungsaspektes gemachten Ausführungen Bezug genommen. Die dort in Bezug auf die transpirationshemmende Zusammensetzung, insbesondere deren Inhaltsstoffe, gemachten Ausführungen gelten auch für das erfindungsgemäße kosmetische Produkt.

Weitere Merkmale und Vorteile der Erfindung ergeben sich aus der nachfolgenden Beschreibung bevorzugter Ausführungsformen in Form von Beispielen.

### Beispiele

Es wurden mehrere erfindungsgemäße Zusammensetzungen hergestellt und im Hinblick auf ihre transpirationshemmende Wirkung an mehreren Probanden getestet. Die verwendeten Zusammensetzungen sowie die hierbei erhaltenen Ergebnisse sind in den nachfolgenden Tabellen 1 und 2 aufgeführt.

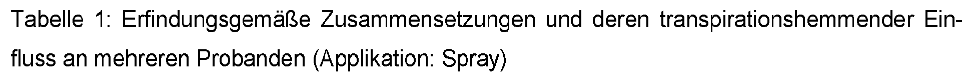

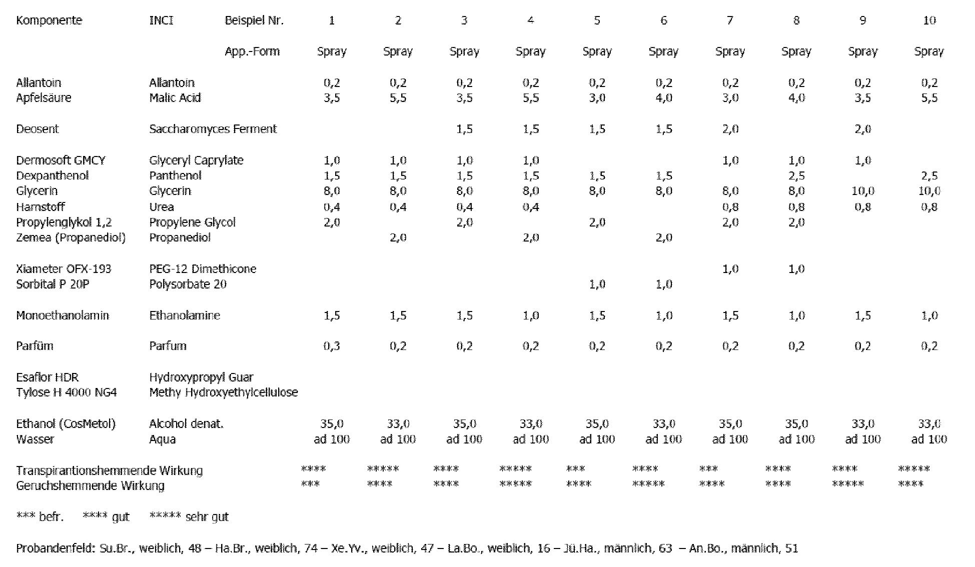

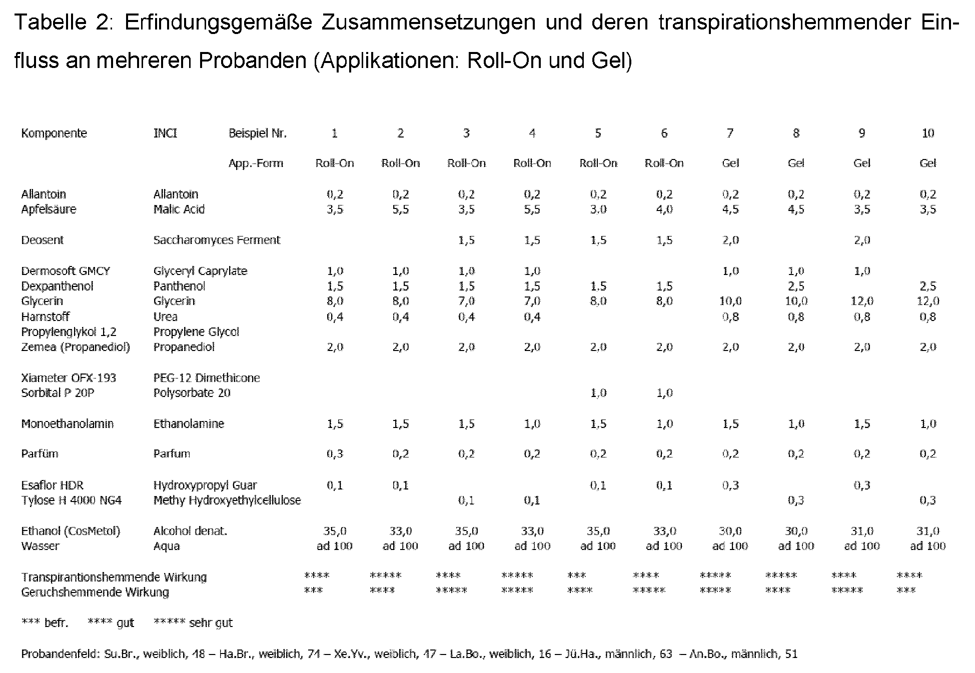

## Patentansprüche

1. Aluminiumsalzfreie, transpirationshemmende Zusammensetzung, enthaltend
- einen transpirationshemmenden Wirkstoff,
- einen pH-Regulator, ausgenommen Triethanolamin,
- einen flüssigen Träger und
- Wasser,
**dadurch gekennzeichnet, dass** es sich bei dem transpirationshemmenden Wirkstoff um Äpfelsäure und/oder ein Salz davon handelt und der flüssige Träger einen Anteil von höchstens 50 Gew.-% aufweist, bezogen auf das Gesamtgewicht der Zusammensetzung, wobei es sich bei dem flüssigen Träger um einen Alkohol, ausgewählt aus der Gruppe bestehend aus Ethanol, Isopropanol, 2-Methyl-propanol-2 und Gemische aus zwei oder mehreren der genannten Alkohole, handelt.

2. Zusammensetzung nach Anspruch 1, **dadurch gekennzeichnet, dass** die Äpfelsäure einen Anteil von 1 Gew.-% bis 10 Gew.-%, insbesondere 2 Gew.-% bis 8 Gew.-%, vorzugsweise 3 Gew.-% bis 5 Gew.-%, aufweist, bezogen auf das Gesamtgewicht der Zusammensetzung.

3. Zusammensetzung nach Anspruch 1 oder 2, **dadurch gekennzeichnet, dass** der pH-Regulator ausgewählt ist aus der Gruppe bestehend aus Monoethanolamin, Diethanolamin und ein Gemisch aus Monoethanolamin und Diethanolamin.

4. Zusammensetzung nach einem der vorhergehenden Ansprüche, **dadurch gekennzeichnet, dass** der pH-Regulator einen Anteil von 0.25 Gew.-% bis 5 Gew.-%, insbesondere 0.5 Gew.-% bis 4 Gew.-%, vorzugsweise 0.75 Gew.-% bis 1.75 Gew.-%, aufweist, bezogen auf das Gesamtgewicht der Zusammensetzung.

5. Zusammensetzung nach einem der vorhergehenden Ansprüche, **dadurch gekennzeichnet, dass** der flüssige Träger einen Anteil von 20 Gew.-% bis 50 Gew.-%, insbesondere 25 Gew.-% bis 45 Gew.-%, vorzugsweise 30 Gew.-% bis 40 Gew.-%, aufweist, bezogen auf das Gesamtgewicht der Zusammensetzung.

6. Zusammensetzung nach einem der vorhergehenden Ansprüche, **dadurch gekennzeichnet, dass** die Zusammensetzung einen Wasseranteil von 10 Gew.-% bis 80 Gew.-%, insbesondere 20 Gew.-% bis 75 Gew.-%, vorzugsweise 30 Gew.-% bis 65 Gew.-%, aufweist, bezogen auf das Gesamtgewicht der Zusammensetzung.

7. Zusammensetzung nach einem der vorhergehenden Ansprüche, **dadurch gekennzeichnet, dass** die Zusammensetzung ferner Saccharomyces Ferment enthält.

8. Zusammensetzung nach Anspruch 7, **dadurch gekennzeichnet, dass** das Saccharomyces Ferment einen Anteil von 0.5 Gew.-% bis 5 Gew.-%, insbesondere 1 Gew.-% bis 3.5 Gew.-%, vorzugsweise 1.25 Gew.-% bis 2.5 Gew.-%, aufweist, bezogen auf das Gesamtgewicht der Zusammensetzung.

9. Zusammensetzung nach einem der vorhergehenden Ansprüche, **dadurch gekennzeichnet, dass** sie auf 100 Gew.-% folgende Inhaltsstoffe enthält:
- 1 Gew.-% bis 11 Gew.-%, insbesondere 2 Gew.-% bis 8 Gew.-%, vorzugsweise 3 Gew.-% bis 6.5 Gew.-%, Apfelsäure,
- 0.25 Gew.-% bis 5 Gew.-%, insbesondere 0.5 Gew.-% bis 4 Gew.-%, vorzugsweise 0.75 Gew.-% bis 1.75 Gew.-%, eines pH-Regulators,
- 20 Gew.-% bis 50 Gew.-%, insbesondere 25 Gew.-% bis 45 Gew.-%, vorzugsweise 30 Gew.-% bis 40 Gew.-%, des flüssigen Trägers,
- 10 Gew.-% bis 80 Gew.-%, insbesondere 20 Gew.-% bis 75 Gew.-%, vorzugsweise 30 Gew.-% bis 65 Gew.-%, Wasser.
- 0.5 Gew.-% bis 5 Gew.-%, insbesondere 1 Gew.-% bis 3.5 Gew.-%, vorzugsweise 1.25 Gew.-% bis 2.5 Gew.-%, Saccharomyces Ferment
- 0.25 Gew.-% bis 5 Gew.-%, insbesondere 0.5 Gew.-% bis 4 Gew.-%, vorzugsweise 0.75 Gew.-% bis 3 Gew.-%, eines Komplexbildners,
- 0.01 Gew.-% bis 1 Gew.-%, insbesondere 0.05 Gew.-% bis 0.75 Gew.-%, vorzugsweise 0.1 Gew.-% bis 0.5 Gew.-%, eines Viskositätsregulators bzw. Filmbildners,
- 0.1 Gew.-% bis 2.5 Gew.-%, insbesondere 0.25 Gew.-% bis 2 Gew.-%, vorzugsweise 0.5 Gew.-% bis 1.5 Gew.-%, eines Tensids
- 2 Gew.-% bis 25 Gew.-%, insbesondere 3.5 Gew.-% bis 20.5 Gew.-%, vorzugsweise 4.5 Gew.-% bis 18.5 Gew.-%, eines Hautpflegestoff-Komplexes,
- 0.05 Gew.-% bis 2.5 Gew.-%, insbesondere 0.1 Gew.-% bis 1.5 Gew.-%, vorzugsweise 0.2 Gew.-% bis 1 Gew.-%, eines Duftstoffes,

10. Zusammensetzung nach einem der vorhergehenden Ansprüche, **dadurch gekennzeichnet, dass** die Zusammensetzung als Lösung, Dispersion, Suspension, Gel, wie Hydrogel, Creme, Paste, Salbe, Roll-on-Zusammensetzung, Stift, Spray-Sud, Pumpspray oder in Kombination mit einem Treibmittel als Aerosolspray konfektioniert ist.

11. Kosmetisches Produkt, enthaltend oder bestehend aus einer Zusammensetzung nach einem der vorhergehenden Ansprüche.

## Claims

1. A perspiration-inhibiting composition free of aluminum salt, comprising
- an active perspiration-inhibiting ingredient,
- a pH regulator, except for triethanolamine,
- a liquid carrier, and
- water,
**characterized in that** the active perspiration-inhibiting ingredient is malic acid and/or a salt thereof, and the liquid carrier has a fraction of at most 50 wt%, based on the total weight of the composition, wherein the liquid carrier is an alcohol, selected from the group consisting of ethanol, isopropanol, 2-methylpropan-2-ol, and mixtures of two or more of said alcohols.

2. The composition as claimed in claim 1, **characterized in that** the malic acid has a fraction of 1 wt% to 10 wt%, more particularly 2 wt% to 8 wt%, preferably 3 wt% to 5 wt%, based on the total weight of the composition.

3. The composition as claimed in claim 1 or 2, **characterized in that** the pH regulator is selected from the group consisting of monoethanolamine, diethanolamine and a mixture of monoethanolamine and diethanolamine.

4. The composition as claimed in any one of the preceding claims, **characterized in that** the pH regulator has a fraction of 0.25 wt% to 5 wt%, more particularly 0.5 wt% to 4 wt%, preferably 0.75 wt% to 1.75 wt%, based on the total weight of the composition.

5. The composition as claimed in any one of the preceding claims, **characterized in that** the liquid carrier has a fraction of 20 wt% to 50 wt%, more particularly 25 wt% to 45 wt%, preferably 30 wt% to 40 wt%, based on the total weight of the composition.

6. The composition as claimed in any one of the preceding claims, **characterized in that** the composition has a water fraction of 10 wt% to 80 wt%, more particularly 20 wt% to 75 wt%, preferably 30 wt% to 65 wt%, based on the total weight of the composition.

7. The composition as claimed in any one of the preceding claims, **characterized in that** the composition further comprises Saccharomyces ferment.

8. The composition as claimed in claim 7, **characterized in that** the Saccharomyces ferment has a fraction of 0.5 wt% to 5 wt%, more particularly 1 wt% to 3.5 wt%, preferably 1.25 wt% to 2.5 wt%, based on the total weight of the composition.

9. The composition as claimed in any one of the preceding claims, **characterized in that** it contains to 100 wt% the following ingredients:
- 1 wt% to 11 wt%, more particularly 2 wt% to 8 wt%, preferably 3 wt% to 6.5 wt%, of malic acid,
- 0.25 wt% to 5 wt%, more particularly 0.5 wt% to 4 wt%, preferably 0.75 wt% to 1.75 wt%, of a pH regulator,
- 20 wt% to 50 wt%, more particularly 25 wt% to 45 wt%, preferably 30 wt% to 40 wt%, of the liquid carrier,
- 10 wt% to 80 wt%, more particularly 20 wt% to 75 wt%, preferably 30 wt% to 65 wt%, of water,
- 0.5 wt% to 5 wt%, more particularly 1 wt% to 3.5 wt%, preferably 1.25 wt% to 2.5 wt%, of Saccharomyces ferment,
- 0.25 wt% to 5 wt%, more particularly 0.5 wt% to 4 wt%, preferably 0.75 wt% to 3 wt%, of a complexing agent,
- 0.01 wt% to 1 wt%, more particularly 0.05 wt% to 0.75 wt%, preferably 0.1 wt% to 0.5 wt%, of a viscosity regulator and/or film-former,
- 0.1 wt% to 2.5 wt%, more particularly 0.25 wt% to 2 wt%, preferably 0.5 wt% to 1.5 wt%, of a surfactant,
- 2 wt% to 25 wt%, more particularly 3.5 wt% to 20.5 wt%, preferably 4.5 wt% to 18.5 wt%, of a skincare-substance complex,
- 0.05 wt% to 2.5 wt%, more particularly 0.1 wt% to 1.5 wt%, preferably 0.2 wt% to 1 wt%, of a fragrance.

10. The composition as claimed in any one of the preceding claims, **characterized in that** the composition is formulated as a solution, dispersion, suspension, gel such as hydrogel, cream, paste, salve, roll-on composition, stick, spray sud, pump spray or, in combination with a propellant, as an aerosol spray.

11. A cosmetic product, comprising or consisting of a composition as claimed in any one of the preceding claims.

## Revendications

1. Composition antitranspirante, exempte de sels d'aluminium, contenant :
- un agent actif antitranspirant,
- un régulateur de pH, à l'exception de la triéthanolamine,
- un véhicule liquide et
- de l'eau,
**caractérisée en ce que** l'agent actif antitranspirant consiste en de l'acide malique et/ou un sel de celui-ci, et le véhicule liquide présente une proportion d'au plus 50 % en poids, par rapport au poids total de la composition, le véhicule liquide consistant en un alcool, choisi dans le groupe constitué par l'éthanol, l'isopropanol, le 2-méthyl-propanol-2 et les mélanges de deux ou davantage des alcools mentionnés.

2. Composition selon la revendication 1, **caractérisée en ce que** l'acide malique présente une proportion de 1 % en poids à 10 % en poids, notamment de 2 % en poids à 8 % en poids, de préférence de 3 % en poids à 5 % en poids, par rapport au poids total de la composition.

3. Composition selon la revendication 1 ou 2, **caractérisée en ce que** le régulateur de pH est choisi dans le groupe constitué par la monoéthanolamine, la diéthanolamine et un mélange de monoéthanolamine et de diéthanolamine.

4. Composition selon l'une quelconque des revendications précédentes, **caractérisée en ce que** le régulateur de pH présente une proportion de 0,25 % en poids à 5 % en poids, notamment de 0,5 % en poids à 4 % en poids, de préférence de 0,75 % en poids à 1,75 % en poids, par rapport au poids total de la composition.

5. Composition selon l'une quelconque des revendications précédentes, **caractérisée en ce que** le véhicule liquide présente une proportion de 20 % en poids à 50 % en poids, notamment de 25 % en poids à 45 % en poids, de préférence de 30 % en poids à 40 % en poids, par rapport au poids total de la composition.

6. Composition selon l'une quelconque des revendications précédentes, **caractérisée en ce que** la composition présente une proportion d'eau de 10 % en poids à 80 % en poids, notamment de 20 % en poids à 75 % en poids, de préférence de 30 % en poids à 65 % en poids, par rapport au poids total de la composition.

7. Composition selon l'une quelconque des revendications précédentes, **caractérisée en ce que** la composition contient en outre Saccharomyces Ferment.

8. Composition selon la revendication 7, **caractérisée en ce que** le Saccharomyces Ferment présente une proportion de 0,5 % en poids à 5 % en poids, notamment de 1 % en poids à 3,5 % en poids, de préférence de 1,25 % en poids à 2,5 % en poids, par rapport au poids total de la composition.

9. Composition selon l'une quelconque des revendications précédentes, **caractérisée en ce qu'**elle contient pour 100 % en poids les constituants suivants :
- 1 % en poids à 11 % en poids, notamment 2 % en poids à 8 % en poids, de préférence 3 % en poids à 6,5 % en poids, d'acide malique,
- 0,25 % en poids à 5 % en poids, notamment 0,5 % en poids à 4 % en poids, de préférence 0,75 % en poids à 1,75 % en poids, d'un régulateur de pH,
- 20 % en poids à 50 % en poids, notamment 25 % en poids à 45 % en poids, de préférence 30 % en poids à 40 % en poids, du véhicule liquide,
- 10 % en poids à 80 % en poids, notamment 20 % en poids à 75 % en poids, de préférence 30 % en poids à 65 % en poids, d'eau,
- 0,5 % en poids à 5 % en poids, notamment 1 % en poids à 3,5 % en poids, de préférence 1,25 % en poids à 2,5 % en poids, de Saccharomyces Ferment,
- 0,25 % en poids à 5 % en poids, notamment 0,5 % en poids à 4 % en poids, de préférence 0,75 % en poids à 3 % en poids, d'un complexant,
- 0,01 % en poids à 1 % en poids, notamment 0,05 % en poids à 0,75 % en poids, de préférence 0,1 % en poids à 0,5 % en poids, d'un régulateur de viscosité ou agent filmogène,
- 0,1 % en poids à 2,5 % en poids, notamment 0,25 % en poids à 2 % en poids, de préférence 0,5 % en poids à 1,5 % en poids, d'un tensioactif,
- 2 % en poids à 25 % en poids, notamment 3,5 % en poids à 20,5 % en poids, de préférence 4,5 % en poids à 18,5 % en poids, d'un complexe de matière pour le soin de la peau,
- 0,05 % en poids à 2,5 % en poids, notamment 0,1 % en poids à 1,5 % en poids, de préférence 0,2 % en poids à 1 % en poids, d'un parfum.

10. Composition selon l'une quelconque des revendications précédentes, **caractérisée en ce que** la composition est conditionnée sous la forme d'une solution, d'une dispersion, d'une suspension, d'un gel, tel qu'un hydrogel, d'une crème, d'une pâte, d'un onguent, d'une composition à bille, d'un bâton, d'un bouillon de pulvérisation, d'une pulvérisation à pompe ou en combinaison avec un agent gonflant sous la forme d'une pulvérisation d'aérosol.

11. Produit cosmétique, contenant ou constitué par une composition selon l'une quelconque des revendications précédentes.
